# EUROPEAN PATENT APPLICATION

(11) **EP 0 882 459 A1**
(43) Date of publication of application: **09.12.1998**
(21) Application number: 97500132.2
(22) Date of filing: 04.08.1997
(51) Int. Cl.: A61L 9/03, A01M 1/20

(54) **Conditioning device**

(30) Priority: 03.06.1997 ES 9701552
(71) Applicant: Bolos Bru, Ramon, 46015 Valencia (ES)
(72) Inventor: Bolos Bru, Ramon, 46015 Valencia (ES)
(74) Representative: Davila Baz, Angel

(57) **Abstract**

A conditioning device, including a casing which internally defines a lower chamber (1) with mechanisms and an upper horizontal passage (2) for air circulation whose lower chamber (1) houses an electric micromotor (7), a panel (6) with a temporizing circuit and has mounted on its wall a plug for electric connection (3) and a switch (4); and whose upper passage (2) houses a fan (8) with a vertical spindle (9) that may be activated by means of the electric micromotor and having an upper aspiration mouth (10) located on top of the fan (8), a lateral impulsion mouth (11) and a lower hole (12) situated between the fan (8) and the front impulsion mouth (11), said hole (12) being externally surrounded by a wall (13) to which an exchangeable vessel (14) with the conditioning product and the evaporation wick (15) is connected. The latter (15) is introduced through the mentioned hole (12) and partially crosses the upper horizontal passage (2).

## Description

This invention refers to a device which achieves optimum conditioning as from a conditioning substance which evaporates.

More specifically, the invention is the type consisting of a casing where a fan is housed accelerating the evaporation of the conditioning substance.

The conditioning substance may be of any type, including, for example, the odorant, deodorant, environment purifying types, etc.

In devices of the type indicated above, the fan creates an air current which impinges or acts directly over the conditioning substance, which could provoke excessive evaporation, being impossible to control easily and which, moreover, does not allow a uniform conditioning in the entire volume of the room where the device is located.

The object of this invention is a conditioning device, made in such a way, that it permits an easily controllable and efficient conditioning without enhancing the premature consumption of the conditioning substance.

Another object of the invention is that the conditioning of device has a reduced volume and is easy to install.

A further object of the invention is to obtain a conditioning device in which the conditioning substance is held in an easily exchangeable vessel, permitting replacement, once its contents have been consumed.

The conditioning device, purpose of the invention, consists of a casing which internally defines a lower chamber with mechanisms and an upper horizontal air circulation passage.

The lower chamber houses an electric micromotor, a panel with a temporizing circuit and on its wall, there is an electric connection plug and a switch

On the other hand, the upper passage houses a vertical spindle fan, activated by the electric motor housed in the lower chamber and an upper aspiration mouth, on top of the fan, a lateral impulsion mouth and a lower hole located between the fan and the front impulsion mouth. This lower hole is externally surrounded by a wall connected to an exchangeable vessel, containing a conditioning product and an evaporation wick, introduced into the mentioned hole, such that it partially crosses the upper passage.

The features of the device of the invention are shown below in greater detail with the help of the attached drawings, where an illustrative but not limitative example of embodiment is presented.

Of the drawings:

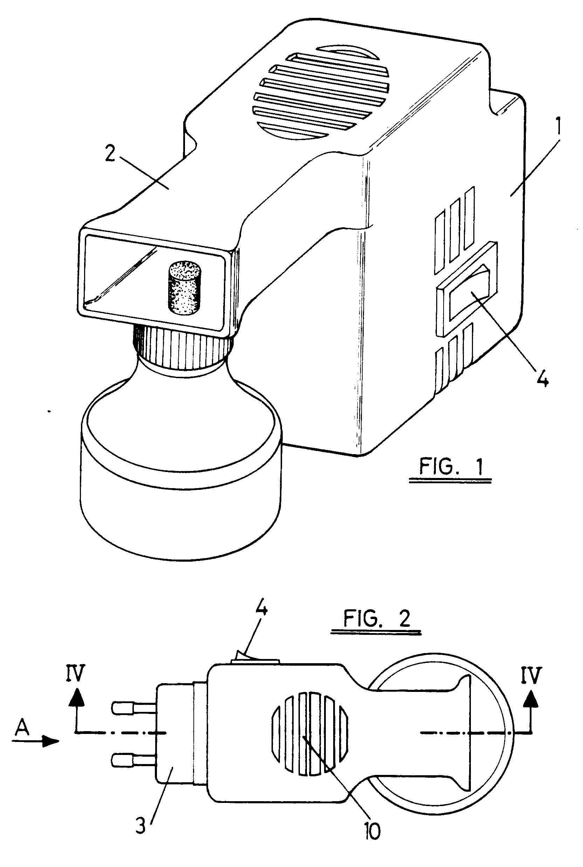

Figure 1 is a front perspective of a conditioning device built according to the invention.
Figure 2 is an upper view of the device shown in Figure 1.
Figure 3 is a rear view of the device, according to direction A of Figure 2.
Figure 4 is a longitudinal section of the device, taken following the line IV-IV of Figure 2.

The device represented in the drawings includes a casing which defines a lower chamber, generally referred to by 1 and an upper horizontal passage 2.

On the wall of this casing, corresponding with the lower chamber 1, there is a plug 3 and a switch 4. The upper horizontal passage 2 protrudes laterally with respect to the lower chamber 1, in an opposite sense to that occupied by plug 3.

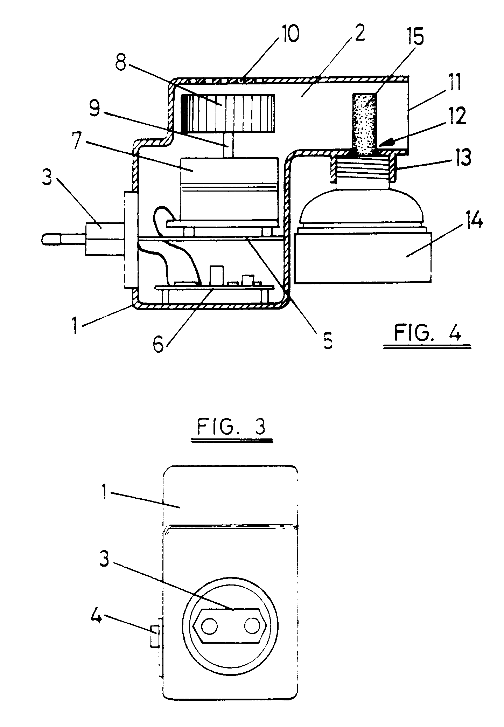

As may be better appreciated in Figure 4, two panels 5 and 6, are fixed inside the lower chamber 1. On the first of these panels, an electric motor 7 is installed, whilst the second panel holds an electric circuit with a temporizer, to supply and control and the motor 7, as from the electric plug 3.

In the upper horizontal passage 2, there is a fan 8, whose spindle 9, is vertical and coaxial with that of the motor 7, being connected to the latter such that the fan is supported by the motor 7. On top of the fan 8, the passage wall 1, has a grille 10, defining an aspiration mouth. The extreme front section 11, of the passage 2, is open, to form a lateral impulsion mouth. On the lower wall, the passage 2 has, between the fan 8 and the impulsion mouth 11, a hole 12, externally surrounded by a wall 13 to which a vessel 14 may be connected and which holds a liquid conditioning substance, this vessel having an absorbent wick 15, which protrudes at the top and may be introduced through the hole 12 of passage 2, to partially cross said passage. The wall 13, externally surrounding the hole 12, will posses the means to permit easy and fast installation and separation of the vessel 14, possibly consisting of an internal or external screw thread.

With the design mentioned above, when connecting the device to a power supply, by means of the plug 3, and activating the switch 4, the fan 8 starts up and aspires air through the grille 10 and impels it along the upper horizontal passage 2, acting over the absorbent wick 15, to finally leave through the impulsion mouth 11.

The action of the air on the absorbent wick 15 accelerates the evaporation of the conditioning substance rising through the wick 15, achieving a rapid but controlled conditioning effect, since the air current does not directly impinge on the conditioning liquid contained in the vessel 14, but over the wick 15, through which the liquid rises in a controlled manner due to capillarity.

The invention may be connected to any electric power supply of a room or premises thanks to plug 3. Moreover, the device may be left plugged in and disconnected thanks to the switch 4.

The circuit mounted on the panel 6, includes a temporizer, such that operation of the motor 7, will take place at pre-fixed intervals of time.

A further advantage of the invention is that when the air supplied by the fan 8 acts on the wick 15, and intimate mixture of the conditioning substance is obtained with the air and is impelled by the mouth 11, easily extending to and reaching the entire volume of the area where the device is located.

## Claims

1. A conditioning device, characterized in that it consists of a casing which internally defines a lower chamber (1) with mechanisms and an upper horizontal passage (2) for air circulation; whose lower chamber (1) houses and electric micromotor (7), a panel (6) with a temporizing circuit and has mounted to its wall an electric plug (3) and a switch (4); an whose upper passage (2) houses a fan (8) with a vertical spindle (9) which may be activated by the electric micromotor (7) and having an upper aspiration mouth (10) located on top of the fan (8), a lateral impulsion mouth (11) and a lower hole (12) situated between the fan (8) and the front impulsion mouth (11), said hole (12) being externally surrounded by a wall (13) to which an exchangeable vessel (14) is connected and containing a conditioning product and an evaporation wick (15) which is introduced through the mentioned hole (12) and partially crosses the upper horizontal passage (2).

2. A device according to claim 1, characterized in that the lower chamber (1) two horizontal panels are fixed, an upper one (5) over which the electric motor is installed and a lower one (6) holding the electric circuit with the temporizer, for motor (7) supply and control, the fan (8) being located on top of the motor in a coaxial position and supported by it.

3. A device according to claim 1, characterized in that the upper horizontal passage (2) protrudes laterally on one side with respect to the lower body (1), in an opposite sense to that occupied by the plug (3), in a position that emerges from the front impulsion mouth (11) in which the hole (12) is located for the absorbent wick (15) and the external wall (14) where the exchangeable vessel (14) containing the conditioning product is coupled.
